# EUROPÄISCHE PATENTANMELDUNG

(11) **EP 1 086 691 A1**
(43) Veröffentlichungstag der Anmeldung: **28.03.2001**
(21) Anmeldenummer: 99104018.9
(22) Anmeldetag: 12.03.1999
(51) Int. Cl.: A61K 9/20, A61K 31/715, A23L 1/09

(54) **Lutschtablette für die Kohlenhydratversorgung mit Langzeitwirkung**

(30) Priorität: 14.03.1998 DE 19811167
(71) Anmelder: findusFit Sportdiätetika GmbH, 94336 Hunderdorf (DE)
(72) Erfinder: SIRTL, Thomas, D-94334 Wiesengelden (DE)

(57) **Zusammenfassung**

Tablette bzw. Lutschtablette zur Kohlenhydratversorgung mit Langzeitwirkung, die aus Maltodextrin (Langzeitkohlenhydrat), Orangenschalenpulver (tonisierend, magenberuhigend), B- Vitaminen (Kohlenhydratstoffwechsel), Magnesiumcitrat (Muskeltätigkeit) besteht und sich hervorragend zur Langzeitkohlenhydratversorgung z.B. von Ausdauersportlern eignet, da die Resorption des Kohlenhydrats unter Umgehung des Magens bereits in der Mundhöhle erfolgt und die Verstoffwechselung des Maltodextrins keine Insulinausschüttung bewirkt.

## Beschreibung

Herkömmliche Produkte zur Kohlenhydratversorgung werden den Bedürfnissen von manchen Interessenten, z.B. Ausdauersportlern, nach Kohlenhydraten in komprimierter Form mit Langzeiteffekt ohne Verdauungsenergie zu benötigen nicht gerecht.

Bisher auf dem Markt befindliche Präparate sind in fester Form nur als Traubenzucker erhältlich und haben somit einen Kurzzeitcharakter oder sie sind in fester Form als Riegel erhältlich, die im Magen aufgelöst werden müssen und Fett und Eiweiß enthalten. Weitere Produkte gibt es in flüssiger Form, die ebenfalls über den Magen aufgenommen werden und nicht über die Mundhöhle. Allen Produkten fehlt ebenfalls eine magenberuhigende Komponente, wie in unserem Produkt das enthaltene Orangenschalenpulver.

Zum Stand der Technik gehört es bereits, zur Herstellung von Tabletten Maltodextrine zu verwenden. Diese Maltodextrine werden jedoch lediglich als Träger für die eigentlichen aktiven Substanzen wie Medikamente oder Vitamine eingesetzt (EP 192 460 A2, US 55 27 540, US 57 14 168). Maltodextrin wird im Rahmen des Standes der Technik also nur als preisgünstiges und einfach zu verarbeitendes Hilfsmittel für die Herstellung von Tabletten benutzt, aber nicht selber als aktive Substanz betrachtet bzw. eingesetzt. Die Erfindung beruht dahingegen auf der Erkenntnis, daß Maltodextrin ein idealer Energielieferant insbesondere für Ausdauersportler ist, da Maltodextrin bereits in der Mundhöhle während des Lutschens allmählich resorbiert (d. h. unter Umgehung des Magens) und langsam verstoffwechselt wird. Zum Stand der Technik gehört es auch, Magnesiumcitrat als Zusatzstoff zu verwenden (DE 38 32 638 A1).

Der im Schutzanspruch angegebenen Erfindung liegt das Problem zugrunde, ein Produkt zu schaffen, das eine Langzeitkohlenhydratversorgung sicherstellt, den Magen umgeht und beruhigt, weder Eiweiß noch Fett enthält (kein Energieaufwand für die Verdauung), dafür aber Zusatzstoffe für den Kohlenhydratstoffwechsel und die Muskeltätigkeit enthält. Das Ganze sollte in komprimierter Form verfügbar und leicht in der Handhabung sein.

Dieses Problem wird mit unserer Lutschtablette zur Kohlenhydratversorgung mit Langzeitwirkung, die bereits in der Mundhöhle wirkt und mit spezifisch ausgerichteten Zusätzen kombiniert wird, erreicht.

Mit der Erfindung wird erreicht,
daß Maltodextrine verwendet werden, die langsam verstoffwechselt werden und damit stetig Dextrose für einen erhöhten Blutzuckerspiegel zur Verfügung steht, ohne das eine
   Insulinausschüttung erfolgt,
daß weder Eiweiß noch Fett, die zum Verdauen Energie verbrauchen würden, enthalten sind,
daß die Vitamine B1 und B2 enthalten sind, die zur Kohlenhydratverbrennung wichtig sind,
daß Magnesiumcitrat enthalten ist, um Muskelkrämpfen vorzubeugen,
daß Orangenschalenpulver enthalten ist, um leicht anregend und tonisierend zu wirken und den Magen zu beruhigen.

Ein Ausführungsbeispiel könnte wie folgt zusammengesetzt sein:

| | |
|---|---|
| -Maltodextrin | > 90% |
| -Magnesiumcitrat bis zu | 5% |
| -Orangenschalenpulver bis zu | 3% |
| -Vitamine B1,B2 bis zu | 0,04% |
| -Zusatzstoffe,Hilfsstoffe bis zu | 6% |

Es versteht sich, daß diese Zusammenstzung nur als Beispiel zu verstehen ist, und daß auch andere oder zusätzliche wirksame Substanzen dem Maltodextrin beigemischt werden können.

## Patentansprüche

1. Lutschtablette für die Kohlenhydratversorgung mit Resorption des Kohlenhydrats in der Mundhöhle, wobei die Lutschtablette wenigstens 90% Maltodextrin enthält.

2. Lutschtablette nach Anspruch 1, dadurch gekennzeichnet, daß sie zusätzlich die Vitamine B1 und B2, Magnesiumcitrat und Orangenschalenpulver enthält.

3. Verwendung von Maltodextrin zur Herstellung einer Lutschtablette für die Kohlenhydratversorgung wobei die Resorption des Maltodextrins bereits in der Mundhöhle während des Lutschens erfolgt.

4. Verfahren nach Anspruch 3, dadurch gekennzeichnet, daß der Lutschtablette zusätzlich auch die Vitamine B1 und B2, Magnesiumcitrat und Orangenschalenpulver beigefügt werden.
